(19)

Europäisches
Patentamt
European
Patent Office
Office européen
des brevets

(11) **EP 1 956 095 A1**

(12) **EUROPEAN PATENT APPLICATION**

(43) Date of publication:
13.08.2008  Bulletin 2008/33

(51) Int Cl.:
*C12Q 1/04* (2006.01)          *G01N 33/569* (2006.01)

(21) Application number: 08250438.2

(22) Date of filing: 06.02.2008

(84) Designated Contracting States:
AT BE BG CH CY CZ DE DK EE ES FI FR GB GR
HR HU IE IS IT LI LT LU LV MC MT NL NO PL PT
RO SE SI SK TR
Designated Extension States:
AL BA MK RS

(30) Priority: 06.02.2007  GB 0702278

(71) Applicant: Mercian Science Limited
Unit 8, D2 Maybrook Industrial Park
Maybrook Road, Minworth
Sutton Coldfields, West Midlands B761AL (GB)

(72) Inventors:
• Usher, Johathan,
c/o Mercian Science
Minworth, Sutton Coldfield,
West Midlands B76 1AL (GB)
• Lewis, John,
c/o Mercian Science
Minworth, Sutton Coldfield,
West Midlands B76 1AL (GB)

(74) Representative: Elsy, David et al
Withers & Rogers LLP
Goldings House
2 Hays Lane
London SE1 2HW (GB)

(54) **Legionella Assay**

(57)    The invention provides a method and kit for the detection of *Legionella*.

Fig. 2.

```
              ┌────────────────┐
              │  1 litre Water │
              └───────┬────────┘
                      ▼
          ┌──────────────────────┐
          │ Filter through membrane │
          └───────────┬──────────┘
                      ▼
      ┌────────────────────────────┐
      │ Release bacteria from filter and │
      │      resuspend to 10 ml      │
      └──────────────┬─────────────┘
                      ▼
      ┌────────────────────────────┐
      │ Centrifuge and resuspend in 1 ml │
      └──────────────┬─────────────┘
                      ▼
      ┌────────────────────────────┐
      │ Centrifuge and resuspend in 0.3 ml │
      └──────────────┬─────────────┘
              A       B       C

   ┌──────────────┐ ┌──────────────┐ ┌──────────────┐
   │ Stain 0.1 ml for │ │ Stain 0.1 ml for │ │ Plate 0.1 ml onto │
   │   Serogroup 1   │ │ Serogroups 1 - 15 │ │      Agar       │
   └───────┬────────┘ └───────┬────────┘ └──────────────┘
           ▼                  ▼
   ┌──────────────┐ ┌──────────────┐
   │ Counterstain with │ │ Counterstain with │
   │  viability stain  │ │  viability stain  │
   └──────────────┘ └──────────────┘
```

4 hours

EP 1 956 095 A1

**Description**

*Legionella,* the causative agent of Legionnaires' disease and Pontiac fever can be difficult and time consuming to detect. The time delay between suspecting and detecting an outbreak of *Legionella* can lead to the unnecessary infection of large numbers of people. The present invention provides a rapid, reliable and sensitive assay for *Legionella.*

*Legionella* commonly occurs in natural and artificial water reservoirs, such as water cooling towers, and occurs less often is soil and organic matter. Proliferation of *Legionella* is favoured by water temperatures of between 25°C and 42 °C, the presence of algae or protozoa and calcium or magnesium salt-containing sediments. Common sources of *Legionella* infections include plumbing water, air-conditioning systems, air-moisturising appliances, showers, fountains, spa and other mechanisms producing water mist. Infection occurs through the inhalation of bacteria-laden water droplets, aerosols or dust or by choking. The incubation time of the disease is usually from 2 to 10 days (Szymanska, 2004).

*Legionella* is the causative agent of legionellosis which classically presents as two distinct clinical entities, Legionnaires' disease (a severe multisystem disease involving pneumonia) and Pontiac fever (a self-limited flu-like illness) (Fields, Benson & Besser, 2002).

The Legionellaceae comprise at least 48 species of bacteria with more than 70 serogroups. *Legionella pneumophila* is isolated from 90% or more of culture proven clinical cases with *L. pneumophila* being the most common cause of Legionnaires' disease. *L. pneumophila* is highly pathogenic and has 15 serogroups (Szymanska, 2004, Roig & Rello, 2003). *L. pneumophila* serogroup 1 has been confirmed as the pre-eminent pathogen in studies of community-acquired legionellosis (Roig & Rello, 2003). Immuno-compromised people are particularly susceptible to *Legionella* infection.

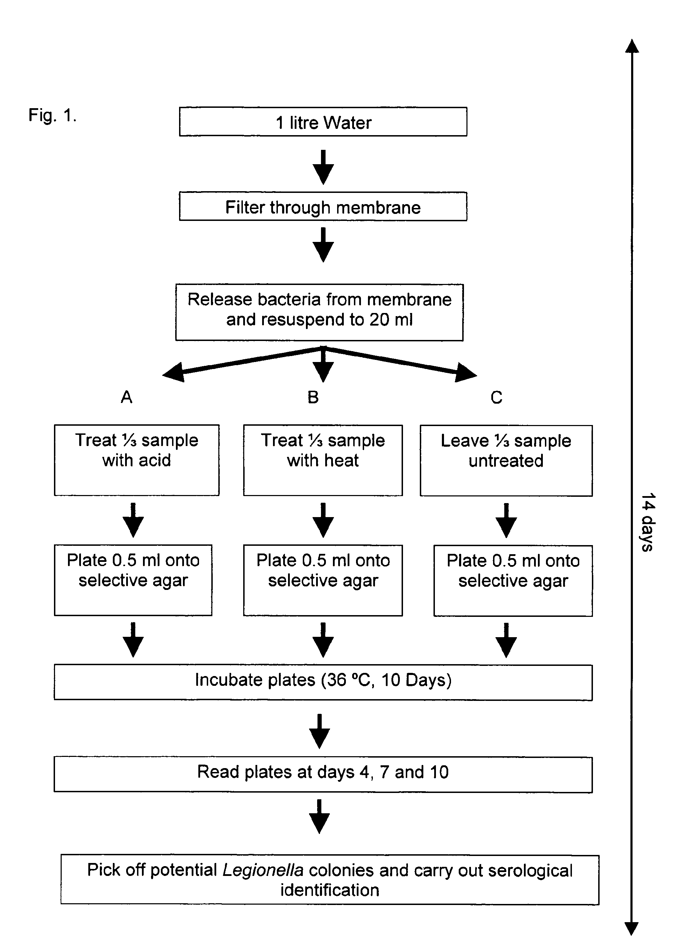

In order to identify, and therefore control, the spread of *Legionella,* it currently is necessary to be able to culture the bacteria from an environmental sample. One current detection method is summarised in Fig. 1.

Typically, a 1 litre sample of water suspected of containing *Legionella* is collected and filtered through a membrane. The bacteria are released from the membrane and resuspended to 20 ml in a suitable liquid. The sample is split into three equal aliquots (A, B, C). Aliquot A is treated with acid. Aliquot B is treated with heat and aliquot C is not treated. 0.5 ml of each of the samples is plated onto agar selective for *Legionella* and incubated for 10 days. Legionella GVPC agar (obtainable from BioMerieux S.A. France) may be used. Agar plates are generally incubated at 36 °C $\pm$ 1 °C (under conditions that will prevent the plates from dehydrating). The plates are examined at days 4, 7 and 10 and putative *Legionella* colonies are picked and subjected to serological identification. Suitable serological identification methods are used. For example, serological identification may be undertaken as follows: place 1 drop serological latex onto a reaction card. Then place 1 drop of diluent buffer onto the same reaction card. Pick off a single suspect colony and emulsify in the buffer ensuring a smooth emulsion is produced. Mix the latex and the buffer together and spread to cover the reaction area on the card. Gently rock the card in a circular motion and look for agglutination. Preferably rocking is carried out for no longer than 1 minute. A positive reaction is seen by an agglutination of the latex particles within 1 minute in the test sample but not in the negative control. A negative reaction is reported if no agglutination is seen.

Current detection methods such as that described in Figure 1 are time consuming and not particularly sensitive. Conventional culture of *Legionella* requires culture of up to 10 days before a negative result can be given and should a *Legionella* colony appear at the 10 day point, if serotyping is required, this may take up to another 4 days. The consequences of such detection methods include the production of false-negative results leading to the incorrect conclusion that *Legionella* are absent from a sample. Furthermore, the length of the test leads to a delay in detection and consequently this can lead to the spread of *Legionella* between the time of collection of a sample and the production of a test result. Such consequences can detrimentally affect public health and consequent closure of public amenities which may have adverse economic effects.

Therefore, what is required is an assay method which overcomes one or more of these disadvantages.

The method of this invention is useful as an initial screen to determine whether a sample contains *Legionella.* The method may also be used to test the effectiveness of cleaning and/or sterilisation following diagnosis of *Legionella* in a sample and attempted eradication of that source of *Legionella.*

The inventors have devised an assay which is fast, sensitive and reliable. Assays to obtain initial results may be carried out in under 24 hours and in as little as 4 hours. Furthermore, the assay may be carried out using mobile equipment, such as equipment which is sufficiently small to be housed in a van. This allows tests to be rapidly carried out on site, away from permanently based laboratories

The inventors have found that their method is capable of detecting as few as one bacterium per litre of sample. This is significantly better than current methods which have a limit of detection of 60 bacteria per litre of water.

A first aspect of the invention provides a method for detecting *Legionella* comprising:

(a) obtaining a sample of liquid suspected of containing *Legionella,*
(b) concentrating the sample to produce a concentrated sample, and
(c) staining an aliquot of the concentrated sample with *Legionella*-specific stain.

The method may include two or more concentration steps. A two stage concentration is preferred in order to sufficiently concentrate the sample so that it can all be examined. This increases the sensitivity of the method and the limit of detection. Ideally, the concentration step provides a 1000-fold to 5000-fold increase in concentration. Concentration may be achieved by any suitable method. For example, material may be captured on a filter and suspended in a relatively small volume of liquid. Another suitable method includes centrifugation to produce a pellet of material followed by suspension of that material in a relatively small volume of liquid. Preferably filtration followed by redispersion in a suitable liquid and then concentrating the liquid by centrifugation, is used. Preferably the method comprises the steps of:

(i) filtering *Legionella* from the sample of liquid with a filter,
(ii) eluting *Legionella* from the filter, and
(iii) concentrating *Legionella* in the eluent by centrifugation.

In one preferred embodiment, an initial sample of, for example, 1 litre is taken and filtered through a membrane. Material caught by the membrane is typically suspended in liquid to 10 ml. The resultant suspension is firstly centrifuged, the supernatant decanted to leave 1 ml and the resultant pellet is typically resuspended in the residual supernatant to 1 ml. The 1 ml suspension is secondly centrifuged and the resultant pellet resuspended in a manner analogous to that described above to 0.3 ml. Therefore, the initial sample is concentrated 3333-fold. The concentration steps may be adapted to achieve a higher or lower extent of concentration.

Preferably the membrane has one or more of the following characteristics:

Material: Nitrocellulose ester
Pore size: Up to 0.45 $\mu$m, preferably 0.22 $\mu$m or 0.45 $\mu$m, most preferably 0.45 $\mu$m.

A particularly preferred membrane is nitrocellulose with 0.45 $\mu$m pore size. Such membranes are commercially available.

Preferably the first centrifugation step is 3000g for 33 $\pm$1 min. Preferably the second centrifugation step is 10 minutes at 6000 RCF (relative centrifugal force). Preferably centrifugation is carried out at room temperature.

A preferred stain comprises labelled antibodies wherein the antibodies are specific for the *Legionella* to be detected. Preferably, the antibodies are monoclonal antibodies. An advantage of using monoclonal antibodies as a carrier of the stain is that they bind only to protein receptors on the target organism and do not cross-react with other organisms.

Preferably the aliquot is not cultured on a culture medium to grow on the bacteria, prior to staining with the *Legionella-specific* stain.

Suitable stains include fluorescent dyes such as fluorescein isothiocyanate (FITC) labelled antibodies. An advantage of this stain is the low interference between the light emitted from the monoclonal stain and the viability stain, thus facilitating carrying out the test. Other stains known for labelling antibodies *per se* may be used. These include visible and non-visible stains such as gold sols, latex beads, horseradish peroxidase and radiolabels.

Since *Legionella pneumophila* is the causative agent of a large number of outbreaks of Legionnaires' disease, preferably the stain is specific for *Legionella pneumophila.* For example, suitable stains include direct fluorescent monoclonal antibodies (DFA) available from Pro-Lab Diagnostics (South Wirral, UK). Such antibodies may be specific for one or more serogroups of *L. pneumophila.* For example, DFA are available for each of serogroups 1 to 14 and appropriate combinations of each DFA would allow any serogroup to be distinguished from any other serogroup. Stains capable of distinguishing two or more strains may be used. One preferred stain is specific for *Legionella pneumophila* serogroup 1, for example *L. pneumophila* serogroup 1 DFA Reagent available from Pro-Lab Diagnostics. Another preferred stain is specific for the group comprising serogroups 1 to 15 of *Legionella pneumophila.* Another preferred stain is specific for the group comprising serogroups 1-14 of *Legionella pneumophila* for example the *L. pneumophila* serogroups 1 - 14 DFA reagent (Pro-Lab Diagnostics). Further useful stains include those which distinguish between one or more of serogroups 2 to 15 of *Legionella pneumophila.*

A further preferred stain is specific for the group comparing serogroups 1 to 14 of *Legionella pneumophila,* for example available from Pro-Lab Diagnostics.

Any suitable method of serotyping may be used. For example one or more of the following may be used: monoclonal antibodies, pulsed filed gel electrophoresis (PFGE), arbitrarily primed polymerase chain reaction (AP-PCR), multi-locus enzyme typing, restriction fragment length polymorphism (RFLP), amplified fragment length polymorphism (AFLP). Such methods are reviewed in (Fields, Benson & Besser, 2002). A preferred staining method comprises:

1. Placing into each of the wells of two microscope slides.
2. Air drying the slide and heating gently for up to 30 minutes on a hot plate to allow the liquid on slide to evaporate to dryness.

3. Fixing the slide in 10% formalin for a minimum of 15 minutes.

4. Draining the formalin and rinsing the slide in distilled deionised water, then air-drying the slide.

5. Adding 10 ml, or sufficient to cover the sample, of the monoclonal FITC stain serogroup 1 and serogroup 1-14.

6. Placing the slides in a moist chamber and incubating at 37 °C for 25 minutes ± 5 minutes.

7. Gently rinsing slides individually with PBS to remove unbound stain.

8. Rinsing slides with deionised water and allowing to air dry. After drying the slides should be mounted and examined without delay.

9. Adding 4 to 5 drops of mounting medium to the slide and applying the coverslip.

10. Examining the slides, preferably under subdued lighting, because the less ambient light present the easier it is to read the slides.

11. Using a fluorescence microscope to examine slides under a low power (x40) objective.

12. If fluorescent rods are observed examining under a high power (xl00) oil immersion objective, to confirm the presence of *L. pneumophila.*

The intensity at which the sample slide fluoresces is scored using a ++ system. With every batch of samples a QC slide of a known *L. pneumophila* serogroup 1 is stained in parallel with the test batch. The intensity at which this slide fluoresces at is considered to be a level of +++ fluorescence. Fluorescence at less than +++ may be attributed to auto-fluorescing material. Hence for a slide to be considered positive the fluorescence must be equal to or greater than that of the control slide.

In a preferred method, a first aliquot of the sample is stained with a stain specific for serogroup 1 of *Legionella pneumophila* and a second aliquot of the sample is stained with a stain specific for the group comprising 2 or more of serogroups 1 to 15 of *Legionella,* preferably all of serogroups 1 to 15 (suitable stains are available from Pro-Lab Diagnostics, as discussed above). Optionally an aliquot may be stained with a viability stain to determine whether the detected *Legionella* is viable or non-viable. Suitable viability stains include propidium iodide (Fluka, Buchs SG, Switzerland). Other suitable stains include acridine orange and methylene blue. For example, an aliquot stained for detection of one or more serogroups of *Legionella pneumophila* may be counterstained with a viability stain. Preferably the fluorescence compared with that of a positive control.

Preferably the viability staining does not require a chemical precursor to be taken into the cell and metabolised in order for viability to be determined. An advantage of this is that is allows dormant, but not dead, cells to be identified. Dormant cells may not metabolise the precursor. Preferably the staining system is quick and effective. Preferably no pretreatment of the sample is required. Preferably the staining represents a simple counterstaining step. Preferably the counter-staining is counter-staining with respect to the antibody staining.

The viability stain preferably comprises propidium iodide. This is differently uptaken by cells depending on their health. Healthy cells do not uptake the stain. Dead cells are stained. This may be determined by looking at the cells under a microscope.

Comparative analysis of the first aliquot stained with a stain specific for serogroup 1 of *Legionella pneumophila* and the second aliquot stained with a stain specific for, for example serogroups 1 to 15 *Legionella pneumophila* enables determination of whether the sample includes *Legionella pneumophila* serogroup 1 *(i.e.* if both aliquots show positive staining) or whether the sample includes only non-serogroup 1 *Legionella pneumophila (i.e.* if the first aliquot shows negative staining and the second aliquot shows positive staining). Such information is useful because *Legionella pneumophila* serogroup 1 has been confirmed as the pre-eminent pathogen in studies of community-acquired legionellosis.

An aliquot may be incubated under conditions suitable for culturing *Legionella.* This enables the viability and/or colony morphology to be determined. Colony morphology is useful in determining the presence of *Legionella pneumophila* and *non-pneumophila* species of *Legionella.* If the results of culturing are used in conjunction with the results of viability staining (see above for details), viable but non-culturable (VNC) organisms may be identified. Preferably the viability stain fluoresces under a different wavelength of light to the serogroup stains. This facilitates analysis of the stained aliquots, such as under a fluorescent microscope in which the wavelength of the light can be easily changed, for example by using different filters. For example, non-viable cells may fluoresce green and non-viable cells may fluoresce red.

The invention also provides a kit for detecting *Legionella* by a method according to any preceding claim comprising (i) a filter for filtering *Legionella* from a sample of liquid and (ii) a *Legionella-specific* stain.

Preferably the stain is as defined above.

The invention also comprises use of a kit or method as disclosed herein to detect *Legionella.* The kit may comprise a *Legionella* strain selected from one or more of serogroups 1, 2, 3, 4, 5, 6, 7, 8, 9, 10, 11, 12, 13, 14 and 15.

The present invention is amenable to being carried out in a mobile unit, such as a van.

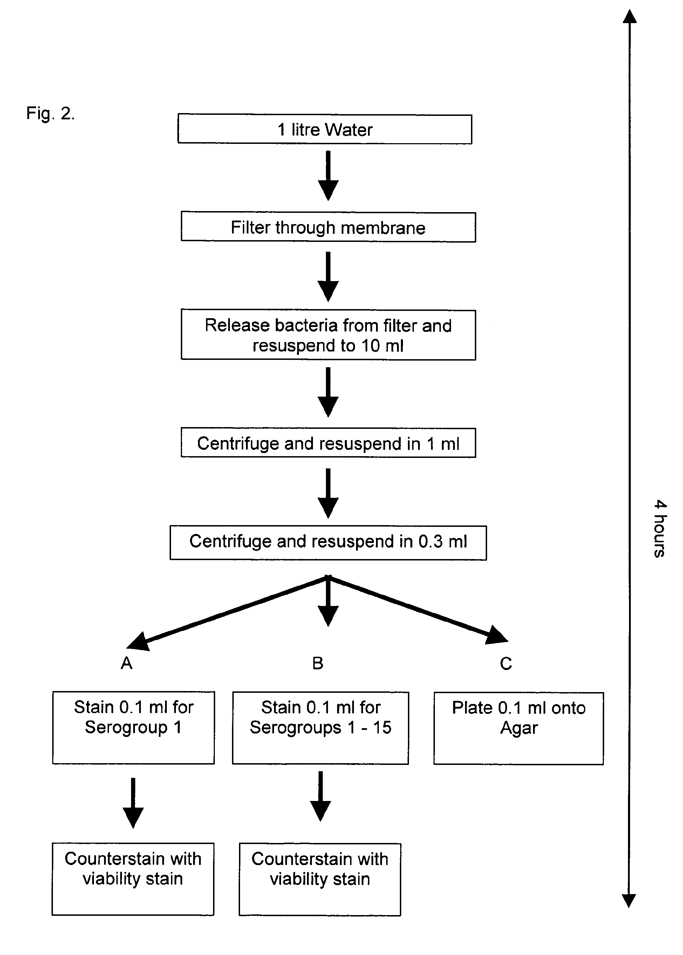

A previously known method for detecting *Legionella* is shown in Figure 1. The method of the present invention will now be explained by way of example only with reference to Figures 2 to 4 in which:

Fig. 1     is a schematic representation of currently known methods of detecting *Legionella.*

Fig. 2.    is a schematic representation of a preferred method of detecting *Legionella* according to the present invention.

Fig. 3.    is a plot of the difference in the paired counts of the culture method (*x*-axis) and the method of the present invention (y-axis). A score of 2 indicates a *Legionella* positive result and a score of 1 indicates *Legionella* negative result.

Fig. 4.    is a summary plot of the frequency (y-axis) of the difference (*x*-axis) in the paired counts of the culture method.

A 1 litre sample of water was taken from a site suspected of being contaminated with *Legionella.* The sample was filtered through a nitrocellulose membrane (pore size: 0.45 $\mu$m). Material caught on the membrane was released from the filter by resuspending the material to 10 ml in sterile 1.40 Ringers solution. The 10 ml suspension was centrifuged. (3000g, 33 $\pm$ 1 min, room temperature). The supernatant was decanted to leave 1 ml and the pellet was resuspended in that of supernatant 1 ml. The 1 ml suspension was centrifuged (6000 RCF, 10 minutes, room temperature) and the pellet resuspended to 0.3 ml in the supernatant. The pellet was split into three 0.1 ml aliquots (A, B, C). Aliquot A was fixed onto a microscope slide (slide A), stained with FITC stain serogroup 1 stain specific for *L. pneumophila* serogroup 1 to detect the absence/presence of *L. pneumophila* serogroup 1 and counterstained with propidium iodide to determine viability of fixed bacteria. Aliquot B was fixed onto a microscope slide (slide B), stained with FITC stain serogroups 1-15 stain (Pro-Lab Diagnostics) specific for *L. pneumophila* serogroups 1 to 15 to detect the absence/presence of *L. pneumophila* serogroup 1 and counterstained with propidium iodide to determine viability of fixed bacteria.

Staining with serogroup specific stains indicated which serogroups of *L. pneumophila* were present in each sample.

Comparison of slide A with slide B indicated whether the sample contained *L. pneumophila* serogroup 1. Samples containing *L. pneumophila* serogroup 1 showed positive staining on slide A and slide B. Samples containing non-serogroup 1 *L. pneumophila* did not show positive staining on slide A and showed positive staining on slide B.

The viability staining on each of slides A and B indicated whether the *Legionella pneumophila* detected were viable (including viable but non-culturable bacteria) or non-viable.

Aliquot C was plated onto agar selective for *L. pneumophila* (BYCE agar, available from Becton Dickinson, Oxford, UK) and incubated at 36 °C for 10 days. BYCE agar is also known as BCYE agar.

BYCE Agar Base (approximate formula per litre):

| | |
|---|---|
| Yeast Extract | 10.0g |
| Ferric Pyrophosphate | 0.5 g |
| ACES* Buffer | 10.0 g |
| Charcoal, Activated | 2.0 g |
| Alpha-Ketoglutarate | 1.0 g |
| Agar | 15.0 g |
| (*                        N-(2-Acetamido)-2-aminoethanesulfonic Acid) | |

To 500 ml of purified water, 2.4 g KOH pellets are added and mixed to dissolve. To this, 38.3 g of BYCE Agar Base powder is added and a further 500 ml water and mixed thoroughly. The mixture is heated with frequent agitation and boiled for 1 minute to completely dissolve the powder. The liquid is then autoclaved at 121 °C for 15 minutes. Following cooling to 45 °C to 50 °C, 4 ml of 10 % filter sterilised solution of L-cysteine HCl is added. This is mixed thoroughly and, if necessary, the pH is adjusted with 1 N HCl or KOH to pH 6.85 $\pm$ 0.1. The resultant mixture is dispensed into Petri dishes with agitation during dispending to keep the charcoal in solution. Samples of the poured plates should be tested for performance using stable, typical control cultures.

It is widely accepted that the average number of positive samples that are expected from *Legionella* samples from the water treatment industry is approximately 6 to 8%. In order to have a higher proportion of positive samples a degree of selection was applied to the samples tested. This was limited to ensuring that samples from sites that were known to yield positive results were included in the validation. This resulted in a significantly higher proportion of positive samples being tested. On no occasion did the method of the present invention falsely identify the non-serogroup 1 *Legionella.*

In order to determine how the new method performed against the standard culture method the two sets of data were statistically compared with the Null Hypothesis being that they were the same. The results of the statistics showed that the sets of data were from different populations. Closer examination of the results shows that the method of the present invention yielded more positive results than the culture method. This confirms that the method of the present invention is more sensitive than conventional methods.

The data generated shows that the method of the present invention performs as well as the conventional culture method when applied to real samples.

Other validation:

In order to test the specific parameters of the method of the present invention, samples were spiked with *Legionella* bacteria and the spiked samples of a range of sample matrixes at both high and low organism numbers were analysed. These tests were undertaken to determine the limit of detection and to determine whether the test could repeatedly give accurate results.

The limit of detection was undertaken by diluting out a suspension of the target organism and determining the lowest concentration at which the organism could still be detected.

Repeatability and reproducibility tests were performed by comparing a cultured aliquot of the spiked sample with results obtained by microscopy. In all cases a pre-determined limit of conformity was set below which the method would be rejected. It was found that in all cases the method performed satisfactorily.

The results of this stage of the validation only lend themselves to basic statistical analysis such as $d^2$. These tests allow a limit of detection to be reported which adequately demonstrates that the method can be accurately and repeatedly undertaken by a range of analysis on a range of sample matrixes.

In addition to the above, and in addition to the data supplied by the manufacturer of the monoclonal antibody, a series of tests were undertaken to determine if there was any apparent cross reactivity with other *Legionella* species or with other organisms commonly found in water samples e.g. *E. coli* and *Pseudomonads.* The results were comparable to those of the manufacturer in that there were no cross-reactions detected.

These tests show that the method of the present invention has a low limit of detection, is reproducible and accurate.

**Data:**

Repeatability / Uncertainty

A series of 10 replicate samples were processed by the method of the present invention and the performance assessed. This process was repeated for a range of sample matrices and at high and low organism numbers and by a number of analysts. The results are shown in Tables 1a to 4b.

| Table 1a: Analyst 1, Tap Water | | | | |
|---|---|---|---|---|
| Sample No | Fluorescence Score | Low Spike Detected/Not Detected | No of organisms detected (No/slide) | |
| 1 | 4+ | Detected | 15 | |
| 2 | 4+ | Detected | 18 | |
| 3 | 4+ | Detected | 8 | |
| 4 | 4+ | Detected | 23 | |
| 5 | 4+ | Detected | 24 | |
| 6 | 4+ | Detected | 10 | |
| 7 | 4+ | Detected | 105 | |
| 8 | 4+ | Detected | 60 | |
| 9 | 4+ | Detected | 55 | |
| 10 | 4+ | Detected | 69 | |
| | | 100% Detected | SD | 30.42 |

| Table 1b: Analyst 1, Tap Water | | | | |
|---|---|---|---|---|
| Sample No | Fluorescence Score | High Spike Detected/Not Detected | No of organisms detected (No/slide) | |
| 1 | 4+ | Detected | 212 | |
| 2 | 4+ | Detected | 193 | |
| 3 | 4+ | Detected | 170 | |
| 4 | 4+ | Detected | 149 | |
| 5 | 4+ | Detected | 226 | |
| 6 | 4+ | Detected | 231 | |
| 7 | 4+ | Detected | 203 | |
| 8 | 4+ | Detected | 263 | |
| 9 | 4+ | Detected | 184 | |
| 10 | 4+ | Detected | 162 | |
| | | 100% Detected | SD | 33.17 |

| Table 2a: Analyst 1, Cooling Tower Water | | | | |
|---|---|---|---|---|
| Sample No | Fluorescence Score | Low Spike Detected/Not Detected | No of organisms detected (No/slide) | |
| 1 | 4+ | Detected | 40 | |
| 2 | 4+ | Detected | 21 | |
| 3 | 4+ | Detected | 37 | |
| 4 | 4+ | Detected | 93 | |
| 5 | 4+ | Detected | 53 | |
| 6 | 4+ | Detected | 60 | |
| 7 | 4+ | Detected | 18 | |
| 8 | 4+ | Detected | 49 | |
| 9 | 4+ | Detected | 58 | |
| 10 | 4+ | Detected | 64 | |
| | | 100% Detected | SD | 20.9 |

| Table 2b: Analyst 1, Cooling Tower Water | | | |
|---|---|---|---|
| Sample No | Fluorescence Score | High Spike Detected/Not Detected | No of organisms detected (No/slide) |
| 1 | 4+ | Detected | 200 |
| 2 | 4+ | Detected | 233 |
| 3 | 4+ | Detected | 181 |
| 4 | 4+ | Detected | 198 |
| 5 | 4+ | Detected | 251 |
| 6 | 4+ | Detected | 249 |

(continued)

| Table 2b: Analyst 1, Cooling Tower Water | | | | |
|---|---|---|---|---|
| **Sample No** | **Fluorescence Score** | **High Spike Detected/Not Detected** | **No of organisms detected (No/slide)** | |
| 7 | 4+ | Detected | 174 | |
| 8 | 4+ | Detected | 146 | |
| 9 | 4+ | Detected | 131 | |
| 10 | 4+ | Detected | 253 | |
| | | **100% Detected** | **SD** | 41.9 |

| Table 3a: Analyst 2, Tap Water | | | | |
|---|---|---|---|---|
| **Sample No** | **Fluorescence Score** | **Low Spike Detected/Not Detected** | **No of organisms detected (No/slide)** | |
| 1 | 4+ | Detected | Not Counted | |
| 2 | 4+ | Detected | | |
| 3 | 4+ | Detected | | |
| 4 | 4+ | Detected | | |
| 5 | 4+ | Detected | | |
| 6 | 4+ | Detected | | |
| 7 | 4+ | Detected | | |
| 8 | 4+ | Detected | | |
| 9 | 4+ | Detected | | |
| 10 | 4+ | Detected | | |
| | | **100% Detected** | **Mean** | 20 |

| Table 3b: Analyst 2, Tap Water | | | | |
|---|---|---|---|---|
| **Sample No** | **Fluorescence Score** | **High Spike Detected/Not Detected** | **No of organisms detected (No/slide)** | |
| 1 | 4+ | Detected | | |
| 2 | 4+ | Detected | | |
| 3 | 4+ | Detected | | |
| 4 | 4+ | Detected | | |
| 5 | 4+ | Detected | | |
| 6 | 4+ | Detected | | |
| 7 | 4+ | Detected | | |
| 8 | 4+ | Detected | | |
| 9 | 4+ | Detected | | |
| 10 | 4+ | Detected | | |
| | | **100% Detected** | **Mean** | 252 |

| Table 4a: Analyst 2, Cooling Tower Water | | | | |
|---|---|---|---|---|
| Sample No | Fluorescence Score | Low Spike Detected/Not Detected | No of organisms detected (No/slide) | |
| 1 | 4+ | Detected | | |
| 2 | 4+ | Detected | | |
| 3 | 4+ | Detected | | |
| 4 | 4+ | Detected | | |
| 5 | 4+ | Detected | | |
| 6 | 4+ | Detected | | |
| 7 | 4+ | Detected | | |
| 8 | 4+ | Detected | | |
| 9 | 4+ | Detected | | |
| 10 | 4+ | Detected | | |
| | | 100% Detected | Mean | 20 |

| Table 4b: Analyst 2, Cooling Tower Water | | | | |
|---|---|---|---|---|
| Sample No | Fluorescence Score | High Spike Detected/Not Detected | No of organisms detected (No/slide) | |
| 1 | 4+ | Detected | | |
| 2 | 4+ | Detected | | |
| 3 | 4+ | Detected | | |
| 4 | 4+ | Detected | | |
| 5 | 4+ | Detected | | |
| 6 | 4+ | Detected | | |
| 7 | 4+ | Detected | | |
| 8 | 4+ | Detected | | |
| 9 | 4+ | Detected | | |
| 10 | 4+ | Detected | | |
| | | 100% Detected | Mean | 252 |

These data show that this method repeatedly gives the expected result at both high and low inoculum sizes and in a range of matrices when the analysis is undertaken by a number of analysts. These data show that the method detects organisms on 100% of occasions.

Reproducibility

Ten duplicate samples were analysed and the results compared, the intensity of the fluorescence was scored using a ++ system scoring ++++ if the fluorescence was the same as the positive control, an approximate count of the fluorescent cells present was also made. This test was performed on serogroup 1 and performed at both high and low organism numbers. The results are shown in Tables 5a to 8b.

| Table 5a: Tap Water, Analyst 1, High Spike | | |
|---|---|---|
| **Bottle No** | **Fluorescence Intensity Score** | **No of Bacilli** |
| 1 | ++++ | 83 |
| 2 | ++++ | 120 |
| 3 | ++++ | 27 |
| 4 | ++++ | 26 |
| 5 | ++++ | 28 |
| 6 | ++++ | 190 |
| 7 | ++++ | 61 |
| 8 | ++++ | 83 |
| 9 | ++++ | 165 |
| 10 | ++++ | 94 |

| Table 5b: Tap Water, Analyst 1, Low Spike | | |
|---|---|---|
| **Bottle No** | **Fluorescence Intensity Score** | **No of Bacilli** |
| 1 | ++++ | 20 |
| 2 | ++++ | 9 |
| 3 | ++++ | 7 |
| 4 | ++++ | 11 |
| 5 | ++++ | 24 |
| 6 | ++++ | 13 |
| 7 | ++++ | 17 |
| 8 | ++++ | 45 |
| 9 | ++++ | 38 |
| 10 | ++++ | 7 |

| Table 6a: Cooling Tower Water, Analyst 1, High Spike | | |
|---|---|---|
| **Bottle No** | **Fluorescence Intensity Score** | **No of Bacilli** |
| 1 | +++ | 165 |
| 2 | ++++ | 22 |
| 3 | ++++ | 240 |
| 4 | ++++ | 340 |
| 5 | ++++ | 215 |
| 6 | ++++ | 244 |
| 7 | ++++ | 222 |
| 8 | ++++ | 231 |
| 9 | ND | ND |

(continued)

| Table 6a: Cooling Tower Water, Analyst 1, High Spike | | |
|---|---|---|
| **Bottle No** | **Fluorescence Intensity Score** | **No of Bacilli** |
| 10 | ND | ND |
| ND: Not Determined | | |

| Table 6b: Cooling Tower Water, Analyst 1, Low Spike | | |
|---|---|---|
| **Bottle No** | **Fluorescence Intensity Score** | **No of Bacilli** |
| 1 | ++++ | 38 |
| 2 | ++++ | 60 |
| 3 | ++++ | 10 |
| 4 | ++++ | 71 |
| 5 | ++++ | 84 |
| 6 | ++++ | 46 |
| 7 | ++++ | 30 |
| 8 | ++++ | 22 |
| 9 | ++++ | 48 |
| 10 | ++++ | 18 |

| Table 7a: Tap Water, Analyst 2, High Spike | | |
|---|---|---|
| **Bottle No** | **Fluorescence Intensity Score** | **No of Bacilli** |
| 1 | ++++ | 317 |
| 2 | ++++ | 110 |
| 3 | ++++ | 160 |
| 4 | ++++ | 141 |
| 5 | ++++ | 342 |
| 6 | ++++ | 99 |
| 7 | ++++ | 287 |
| 8 | ++++ | 306 |
| 9 | ++++ | 204 |
| 10 | ++++ | 237 |

| Table 7b: Tap Water, Analyst 2, Low Spike | | |
|---|---|---|
| **Bottle No** | **Fluorescence Intensity Score** | **No of Bacilli** |
| 1 | ++++ | 57 |
| 2 | ++++ | 26 |
| 3 | ++++ | 33 |
| 4 | ++++ | 37 |

(continued)

| Table 7b: Tap Water, Analyst 2, Low Spike | | |
|---|---|---|
| Bottle No | Fluorescence Intensity Score | No of Bacilli |
| 5 | ++++ | 66 |
| 6 | ++++ | 21 |
| 7 | ++++ | 73 |
| 8 | ++++ | 52 |
| 9 | ++++ | 29 |
| 10 | ++++ | 41 |

| Table 8a: Cooling Tower Water, Analyst 2, High Spike | | |
|---|---|---|
| Bottle No | Fluorescence Intensity Score | No of Bacilli |
| 1 | ++++ | 142 |
| 2 | ++++ | 226 |
| 3 | ++++ | 65 |
| 4 | ++++ | 152 |
| 5 | ++++ | 168 |
| 6 | ++++ | 117 |
| 7 | ++++ | 213 |
| 8 | ++++ | 258 |
| 9 | +++ | 184 |
| 10 | ++++ | 106 |

| Table 8b: Cooling Tower Water, Analyst 2, Low Spike | | |
|---|---|---|
| Bottle No | Fluorescence Intensity Score | No of Bacilli |
| 1 | ++++ | 54 |
| 2 | +++ | 94 |
| 3 | ++++ | 25 |
| 4 | ++++ | 31 |
| 5 | ++++ | 75 |
| 6 | +++ | 20 |
| 7 | ++++ | 63 |
| 8 | ++++ | 19 |
| 9 | ++++ | 36 |
| 10 | ++++ | 47 |

The results from Tables 5a to 8b indicate that there is a 100% correlation between the sets of data for both sample matrices, at high and low organism numbers, and that the test is consistent between analysts.

Limit of Detection

A serial dilution of *Legionella pneumophila* serogroup 1 was prepared and subsequently examined by the method of the present invention, to establish the lowest concentration at which the organism could still be detected. The results are shown in Tables 9 to 12.

| Table 9: Analyst 1, Tap Water | | | | |
|---|---|---|---|---|
| Dilution | No cfu/l | FITC | | |
| | | 1 | 2 | 3 |
| Undiluted | 252 | Present | Present | Present |
| $10^{-1}$ | 20 | Present | Present | Present |
| $10^{-2}$ | 3 | Absent | Absent | Absent |
| $10^{-3}$ | 0 | Absent | Absent | Absent |
| $10^{-4}$ | 0 | ND | ND | ND |
| $10^{-5}$ | ND | ND | ND | ND |
| $10^{-6}$ | ND | ND | ND | ND |
| ND: Not Determined | | | | |

| Table 10: Analyst 1, Cooling Tower Water | | | | |
|---|---|---|---|---|
| Dilution | No cfu/l | FITC | | |
| | | 1 | 2 | 3 |
| Undiluted | 252 | Present | Present | Present |
| $10^{-1}$ | 20 | Present | Present | Present |
| $10^{-2}$ | 3 | Absent | Absent | Absent |
| $10^{-3}$ | 0 | Absent | Absent | Absent |
| $10^{-4}$ | 0 | ND | ND | ND |
| $10^{-5}$ | ND | ND | ND | ND |
| $10^{-6}$ | ND | ND | ND | ND |
| ND: Not Determined | | | | |

| Table 11: Analyst 3, Tap Water | | | | |
|---|---|---|---|---|
| Dilution | No cfu/l | FITC | | |
| | | 1 | 2 | 3 |
| Undiluted | 163 | Present | Present | Present |
| $10^{-1}$ | 23 | Present | Present | Present |
| $10^{-2}$ | 0 | Absent | Absent | Absent |
| $10^{-3}$ | 0 | Absent | Absent | Absent |
| $10^{-4}$ | ND | ND | ND | ND |
| $10^{-5}$ | ND | ND | ND | ND |

(continued)

| Table 11: Analyst 3, Tap Water | | | | |
|---|---|---|---|---|
| Dilution | No cfu/l | FITC | | |
| | | 1 | 2 | 3 |
| $10^{-6}$ | ND | ND | ND | ND |
| ND: Not Determined | | | | |

| Table 12: Analyst 3, Cooling Tower Water | | | | |
|---|---|---|---|---|
| Dilution | No cfu/l | FITC | | |
| | | 1 | 2 | 3 |
| Undiluted | 163 | Present | Present | Present |
| $10^{-1}$ | 23 | Present | Present | Present |
| $10^{-2}$ | 0 | Absent | Absent | Absent |
| $10^{-3}$ | 0 | Absent | Absent | Absent |
| $10^{-4}$ | ND | ND | ND | ND |
| $10^{-5}$ | ND | ND | ND | ND |
| $10^{-6}$ | ND | ND | ND | ND |
| ND: Not Determined | | | | |

The data of Tables 9 to 12 show that the method is able to detect organisms at a level of 20 per litre in both tap water and treated water. Hence the limit of detection (LOD) of this method is recorded as 20 colony forming units per litre (cfu/litre).

Recovery Rate

Recovery rate was determined at a high and low organism concentration, and a range of negative samples were also tested to test for false positive fluorescence. This was repeated on a range of sample matrices and performed by a number of analysts. An estimate of the initial number of organisms in the spiking solution was established by direct inoculation onto BCYE agar. The results are shown in Tables 13 to 16.

| Table 13: Analyst 1, Tap Water | | |
|---|---|---|
| Target - Culture | | FITC |
| HIGH Spike | | |
| 93 | | Present |
| 84 | | Present |
| 60 | | Present |
| | | |
| Mean | 79 | Present |
| LOW Spike | | |
| 9 | | Present |
| 14 | | Present |
| 17 | | Present |

(continued)

| LOW Spike | | |
|---|---|---|
| | | |
| Mean | 13 | Present |
| NEGATIVE | | |
| 0 | | Not Detected |
| 0 | | Not Detected |
| 0 | | Not Detected |
| | | |
| Mean | 0 | Not Detected |

| Table 14: Analyst 1: Cooling Tower Water | | |
|---|---|---|
| Target - Culture | | FITC |
| HIGH Spike | | |
| 93 | | Present |
| 84 | | Present |
| 60 | | Present |
| | | |
| Mean | 79 | Present |
| LOW Spike | | |
| 9 | | Present |
| 14 | | Present |
| 17 | | Present |
| | | |
| Mean | 13 | Present |
| NEGATIVE | | |
| 0 | | Not Detected |
| 0 | | Not Detected |
| 0 | | Not Detected |
| | | |
| Mean | 10 | Not Detected |

| Table 15: Analyst 2: Tap Water | | |
|---|---|---|
| Target - Culture | | FITC |
| HIGH Spike | | |
| 65 | | Present |
| 95 | | Present |
| 106 | | Present |

(continued)

| Table 15: Analyst 2: Tap Water | | |
|---|---|---|
| **Target - Culture** | | **FITC** |
| **HIGH Spike** | | |
| | | |
| Mean | 89 | Present |
| **LOW Spike** | | |
| 27 | | Present |
| 25 | | Present |
| 11 | | Present |
| | | |
| Mean | 21 | Present |
| **NEGATIVE** | | |
| 0 | | Not Detected |
| 0 | | Not Detected |
| 0 | | Not Detected |
| | | |
| Mean | 0 | Not Detected |

| 16: Analyst 2: Cooling Tower Water | | |
|---|---|---|
| **Table Target - Culture** | | **FITC** |
| **HIGH Spike** | | |
| 65 | | Present |
| 95 | | Present |
| 106 | | Present |
| | | |
| Mean | 89 | Present |
| **LOW Spike** | | |
| 27 | | Present |
| 25 | | Present |
| 11 | | Present |
| | | |
| Mean | 21 | Present |
| **NEGATIVE** | | |
| 0 | | Not Detected |
| 0 | | Not Detected |
| 0 | | Not Detected |

(continued)

| NEGATIVE | | |
|---|---|---|
| | | |
| Mean | 0 | Not Detected |

The data of Tables 13 to 16 show that at both high and low numbers of organisms and in a range of matrices the method is capable of consistently detecting them. It also shows that no false negative results were obtained.

Specificity

As the method of the preset invention uses specific staining of the target organism, tests were undertaken to investigate any cross-reaction of the staining with other organisms that may commonly be found in the types of sample typically analysed. Specificity was assessed by analysing samples that had previously been spiked with *E. coli* and *Pseudomonas* species, as well as *Legionella pneumophila* serogroup 1, as the target organism. Also real samples were tested that contained non-serogroup 1 *L. pneumophila,* and other *Legionella* species. 'Serogroup' has been abbreviated to 'sgr'. The results are shown in Table 17.

| Table 17: Analyst 2/3 | | |
|---|---|---|
| **Culture** | **FITC Fluorescence Score** | |
| | Tap Water | Cooling Tower |
| Positive Control | ++++ | ++++ |
| *E. coli* | 0 | 0 |
| *Pseudomonas* | + | + |
| *Legionella* sgr1 | ++++ | +++ |
| *E. coli, Pseudomonas* | ++ | 0 |
| *E. coli, Pseudomonas, Legionella* sgr1 | +++ | ++++ |
| *E. coli, Legionella* sgr1 | ++++ | ++++ |
| *Pseudomonas, Legionella* sgr1 | ++++ | +++ |
| | | |
| **Sample No** | **Organism** | **FITC Fluorescence Score** |
| 32819 | Species | + |
| 32820 | Sgr 2-14 | 0 |
| 32822 | Sgr 2-14 | 0 |
| 32824 | Sgr 2-14 | + |
| 32685 | Species | + |
| 32692 | Species | 0 |
| 32815 | Species | 0 |
| 33124 | Species & Sgr 2-14 | + |
| 33299 | Sgr 2-14 | + |

The data of Table 17 show that there appears to be no cross-reactions with *E. coli* or *Pseudomonas aeruginosa* or with any of the non-serogroup 1 *L. pneumophila* or other *Legionella* species tested. *E. coli* and *Pseudomonas aeruginosa* were distinctly different under the microscope, and hence could be readily identified. *P. aeruginosa* did exhibit a very slight fluorescence but was readily excluded as *Legionella* when compared to the control. There appeared to be no significant difference between the different matrices.

Comparison of Real Samples

311 'real' samples were tested in duplicate using the standard culture method and the method of the present invention. The results were compiled and statistically compared to determine if the method of the present invention performed as well as the traditional method (*i.e.* standard culture method). The statistical hypothesis is that the sets of result are the same, this was tested and the detail is shown below.

In order to undertake statistical analysis on the two populations of data, the qualitative presence/absence results have first to be converted to a numeric value. This was achieved by assigning a score of 1 to a negative sample and 2 to a positive sample. This data is then normalised or transformed by calculating the square root for each result. The transformed data is then used for the statistical analysis, *i.e.* the t-test.

| Table 18: Rapid* *Legionella* Comparative Analysis<br>*The 'rapid' method is the method of the present invention | | | | | | |
|---|---|---|---|---|---|---|
| No | Culture Result | Rapid Result | Transformed data (square rooted) | | $Z=x-y$ | $Z^2$ |
| | | | Culture (x) | Rapid (y) | | |
| 1 | 2 | 2 | 1.4 | 1.4 | 0 | 0 |
| 2 | 1 | 1 | 1 | 1 | 0 | 0 |
| 3 | 1 | 1 | 1 | 1 | 0 | 0 |
| 4 | 1 | 1 | 1 | 1 | 0 | 0 |
| 5 | 1 | 1 | 1 | 1 | 0 | 0 |
| 6 | 1 | 1 | 1 | 1 | 0 | 0 |
| 7 | 1 | 1 | 1 | 1 | 0 | 0 |
| 8 | 1 | 1 | 1 | 1 | 0 | 0 |
| 9 | 1 | 1 | 1 | 1 | 0 | 0 |
| 10 | 1 | 2 | 1 | 1.4 | 0.4 | 0.16 |
| 11 | 1 | 1 | 1 | 1 | 0 | 0 |
| 12 | 1 | 1 | 1 | 1 | 0 | 0 |
| 13 | 1 | 1 | 1 | 1 | 0 | 0 |
| 14 | 1 | 1 | 1 | 1 | 0 | 0 |
| 15 | 1 | 1 | 1 | 1 | 0 | 0 |
| 16 | 1 | 1 | 1 | 1 | 0 | 0 |
| 17 | 2 | 2 | 1.4 | 1.4 | 0 | 0 |
| 18 | 1 | 2 | 1 | 1.4 | 0.4 | 0.16 |
| 19 | 1 | 1 | 1 | 1 | 0 | 0 |
| 20 | 1 | 1 | 1 | 1 | 0 | 0 |
| 21 | 1 | 1 | 1 | 1 | 0 | 0 |
| 22 | 1 | 1 | 1 | 1 | 0 | 0 |
| 23 | 1 | 2 | 1 | 1.4 | 0.4 | 0.16 |
| 24 | 1 | 1 | 1 | 1 | 0 | 0 |
| 25 | 1 | 1 | 1 | 1 | 0 | 0 |
| 26 | 1 | 1 | 1 | 1 | 0 | 0 |
| 27 | 1 | 1 | 1 | 1 | 0 | 0 |

(continued)

| Table 18: Rapid* *Legionella* Comparative Analysis *The 'rapid' method is the method of the present invention | | | | | | |
|---|---|---|---|---|---|---|
| No | Culture Result | Rapid Result | Transformed data (square rooted) | | Z=x-y | Z² |
| | | | Culture (x) | Rapid (y) | | |
| 28 | 1 | 1 | 1 | 1 | 0 | 0 |
| 29 | 1 | 1 | 1 | 1 | 0 | 0 |
| 30 | 1 | 1 | 1 | 1 | 0 | 0 |
| 31 | 1 | 1 | 1 | 1 | 0 | 0 |
| 32 | 1 | 1 | 1 | 1 | 0 | 0 |
| 33 | 1 | 1 | 1 | 1 | 0 | 0 |
| 34 | 1 | 1 | 1 | 1 | 0 | 0 |
| 35 | 1 | 1 | 1 | 1 | 0 | 0 |
| 36 | 1 | 2 | 1 | 1.4 | 0.4 | 0.16 |
| 37 | 1 | 1 | 1 | 1 | 0 | 0 |
| 38 | 1 | 1 | 1 | 1 | 0 | 0 |
| 39 | 1 | 1 | 1 | 1 | 0 | 0 |
| 40 | 1 | 1 | 1 | 1 | 0 | 0 |
| 41 | 1 | 1 | 1 | 1 | 0 | 0 |
| 42 | 1 | 1 | 1 | 1 | 0 | 0 |
| 43 | 1 | 1 | 1 | 1 | 0 | 0 |
| 44 | 1 | 1 | 1 | 1 | 0 | 0 |
| 45 | 1 | 1 | 1 | 1 | 0 | 0 |
| 46 | 1 | 1 | 1 | 1 | 0 | 0 |
| 47 | 1 | 1 | 1 | 1 | 0 | 0 |
| 48 | 1 | 1 | 1 | 1 | 0 | 0 |
| 49 | 1 | 1 | 1 | 1 | 0 | 0 |
| 50 | 1 | 1 | 1 | 1 | 0 | 0 |
| 52 | 1 | 1 | 1 | 1 | 0 | 0 |
| 53 | 2 | 2 | 1.4 | 1.4 | 0 | 0 |
| 54 | 1 | 1 | 1 | 1 | 0 | 0 |
| 55 | 1 | 1 | 1 | 1 | 0 | 0 |
| 56 | 1 | 1 | 1 | 1 | 0 | 0 |
| 57 | 1 | 1 | 1 | 1 | 0 | 0 |
| 58 | 2 | 2 | 1.4 | 1.4 | 0 | 0 |
| 59 | 1 | 1 | 1 | 1 | 0 | 0 |
| 60 | 2 | 2 | 1.4 | 1.4 | 0 | 0 |
| 61 | 2 | 2 | 1.4 | 1.4 | 0 | 0 |
| 62 | 2 | 2 | 1.4 | 1.4 | 0 | 0 |
| 63 | 2 | 2 | 1.4 | 1.4 | 0 | 0 |

(continued)

| No | Culture Result | Rapid Result | Transformed data (square rooted) | | Z=x-y | Z² |
|---|---|---|---|---|---|---|
| | | | Culture (x) | Rapid (y) | | |
| 64 | 2 | 2 | 1.4 | 1.4 | 0 | 0 |
| 65 | 2 | 2 | 1.4 | 1.4 | 0 | 0 |
| 66 | 2 | 2 | 1.4 | 1.4 | 0 | 0 |
| 67 | 2 | 2 | 1.4 | 1.4 | 0 | 0 |
| 68 | 2 | 2 | 1.4 | 1.4 | 0 | 0 |
| 69 | 2 | 2 | 1.4 | 1.4 | 0 | 0 |
| 70 | 2 | 2 | 1.4 | 1.4 | 0 | 0 |
| 71 | 2 | 2 | 1.4 | 1.4 | 0 | 0 |
| 72 | 1 | 2 | 1 | 1.4 | 0.4 | 0.16 |
| 73 | 1 | 1 | 1 | 1 | 0 | 0 |
| 74 | 2 | 2 | 1.4 | 1.4 | 0 | 0 |
| 75 | 2 | 2 | 1.4 | 1.4 | 0 | 0 |
| 76 | 2 | 2 | 1.4 | 1.4 | 0 | 0 |
| 77 | 2 | 1 | 1.4 | 1 | 0.4 | 0.16 |
| 78 | 2 | 2 | 1.4 | 1.4 | 0 | 0 |
| 79 | 2 | 2 | 1.4 | 1.4 | 0 | 0 |
| 80 | 2 | 2 | 1.4 | 1.4 | 0 | 0 |
| 81 | 1 | 1 | 1 | 1 | 0 | 0 |
| 82 | 2 | 2 | 1.4 | 1.4 | 0 | 0 |
| 83 | 1 | 1 | 1 | 1 | 0 | 0 |
| 84 | 1 | 1 | 1 | 1 | 0 | 0 |
| 85 | 1 | 1 | 1 | 1 | 0 | 0 |
| 86 | 1 | 1 | 1 | 1 | 0 | 0 |
| 87 | 1 | 1 | 1 | 1 | 0 | 0 |
| 88 | 1 | 1 | 1 | 1 | 0 | 0 |
| 89 | 1 | 1 | 1 | 1 | 0 | 0 |
| 90 | 1 | 1 | 1 | 1 | 0 | 0 |
| 91 | 1 | 1 | 1 | 1 | 0 | 0 |
| 92 | 1 | 1 | 1 | 1 | 0 | 0 |
| 93 | 1 | 1 | 1 | 1 | 0 | 0 |
| 94 | 1 | 1 | 1 | 1 | 0 | 0 |
| 95 | 1 | 1 | 1 | 1 | 0 | 0 |
| 96 | 1 | 1 | 1 | 1 | 0 | 0 |
| 97 | 2 | 2 | 1.4 | 1.4 | 0 | 0 |
| 98 | 1 | 1 | 1 | 1 | 0 | 0 |

Table 18: Rapid* *Legionella* Comparative Analysis
*The 'rapid' method is the method of the present invention

(continued)

| No | Culture Result | Rapid Result | Transformed data (square rooted) | | Z=x-y | Z$^2$ |
|---|---|---|---|---|---|---|
| | | | Culture (x) | Rapid (y) | | |
| 99 | 1 | 1 | 1 | 1 | 0 | 0 |
| 100 | 1 | 1 | 1 | 1 | 0 | 0 |
| 101 | 2 | 2 | 1.4 | 1.4 | 0 | 0 |
| 102 | 1 | 1 | 1 | 1 | 0 | 0 |
| 104 | 1 | 1 | 1 | 1 | 0 | 0 |
| 105 | 1 | 2 | 1 | 1.4 | 0.4 | 0.16 |
| 106 | 1 | 1 | 1 | 1 | 0 | 0 |
| 107 | 1 | 1 | 1 | 1 | 0 | 0 |
| 108 | 1 | 1 | 1 | 1 | 0 | 0 |
| 109 | 2 | 2 | 1.4 | 1.4 | 0 | 0 |
| 110 | 1 | 1 | 1 | 1 | 0 | 0 |
| 111 | 1 | 1 | 1 | 1 | 0 | 0 |
| 112 | 1 | 1 | 1 | 1 | 0 | 0 |
| 113 | 1 | 1 | 1 | 1 | 0 | 0 |
| 114 | 1 | 1 | 1 | 1 | 0 | 0 |
| 115 | 1 | 1 | 1 | 1 | 0 | 0 |
| 116 | 2 | 2 | 1.4 | 1.4 | 0 | 0 |
| 117 | 1 | 2 | 1 | 1.4 | 0.4 | 0.16 |
| 118 | 1 | 2 | 1 | 1.4 | 0.4 | 0.16 |
| 119 | 1 | 1 | 1 | 1 | 0 | 0 |
| 120 | 2 | 2 | 1.4 | 1.4 | 0 | 0 |
| 121 | 1 | 2 | 1 | 1.4 | 0.4 | 0.16 |
| 122 | 2 | 2 | 1.4 | 1.4 | 0 | 0 |
| 123 | 2 | 2 | 1.4 | 1.4 | 0 | 0 |
| 124 | 1 | 1 | 1 | 1 | 0 | 0 |
| 125 | 1 | 1 | 1 | 1 | 0 | 0 |
| 126 | 1 | 1 | 1 | 1 | 0 | 0 |
| 127 | 1 | 1 | 1 | 1 | 0 | 0 |
| 128 | 1 | 1 | 1 | 1 | 0 | 0 |
| 129 | 1 | 1 | 1 | 1 | 0 | 0 |
| 130 | 1 | 1 | 1 | 1 | 0 | 0 |
| 131 | 1 | 1 | | 1 | 0 | 0 |
| 132 | 1 | 1 | 1 | 1 | 0 | 0 |
| 133 | 1 | 1 | 1 | 1 | 0 | 0 |
| 134 | 1 | 1 | 1 | 1 | 0 | 0 |

Table 18: Rapid* *Legionella* Comparative Analysis
*The 'rapid' method is the method of the present invention

(continued)

| No | Culture Result | Rapid Result | Transformed data (square rooted) | | Z=x-y | Z² |
|---|---|---|---|---|---|---|
| | | | Culture (x) | Rapid (y) | | |
| 135 | 1 | 1 | 1 | 1 | 0 | 0 |
| 136 | 1 | 1 | 1 | 1 | 0 | 0 |
| 137 | 1 | 1 | 1 | 1 | 0 | 0 |
| 138 | 1 | 1 | 1 | 1 | 0 | 0 |
| 139 | 1 | 1 | 1 | 1 | 0 | 0 |
| 140 | 1 | 1 | 1 | 1 | 0 | 0 |
| 141 | 1 | 1 | 1 | 1 | 0 | 0 |
| 142 | 1 | 1 | 1 | 1 | 0 | 0 |
| 143 | 1 | 1 | 1 | 1 | 0 | 0 |
| 144 | 1 | 1 | 1 | 1 | 0 | 0 |
| 145 | 1 | 1 | 1 | 1 | 0 | 0 |
| 146 | 1 | 1 | 1 | 1 | 0 | 0 |
| 147 | 1 | 1 | 1 | 1 | 0 | 0 |
| 148 | 1 | 1 | 1 | 1 | 0 | 0 |
| 149 | 1 | 1 | 1 | 1 | 0 | 0 |
| 150 | 1 | 1 | 1 | 1 | 0 | 0 |
| 151 | 2 | 2 | 1.4 | 1.4 | 0 | 0 |
| 152 | 1 | 1 | 1 | 1 | 0 | 0 |
| 153 | 1 | 1 | 1 | 1 | 0 | 0 |
| 154 | 1 | 1 | 1 | 1 | 0 | 0 |
| 155 | 1 | 1 | 1 | 1 | 0 | 0 |
| 156 | 1 | 1 | 1 | 1 | 0 | 0 |
| 157 | 1 | 1 | 1 | 1 | 0 | 0 |
| 158 | 1 | 1 | 1 | 1 | 0 | 0 |
| 159 | 1 | 1 | 1 | 1 | 0 | 0 |
| 160 | 1 | 1 | 1 | 1 | 0 | 0 |
| 161 | 2 | 2 | 1.4 | 1.4 | 0 | 0 |
| 162 | 1 | 1 | 1 | 1 | 0 | 0 |
| 163 | 1 | 1 | 1 | 1 | 0 | 0 |
| 164 | 1 | 1 | 1 | 1 | 0 | 0 |
| 165 | 1 | 1 | 1 | 1 | 0 | 0 |
| 166 | 1 | 1 | 1 | 1 | 0 | 0 |
| 167 | 1 | 1 | 1 | 1 | 0 | 0 |
| 168 | 1 | 1 | 1 | 1 | 0 | 0 |
| 169 | 1 | 1 | 1 | 1 | 0 | 0 |

Table 18: Rapid* *Legionella* Comparative Analysis
*The 'rapid' method is the method of the present invention

(continued)

| No | Culture Result | Rapid Result | Transformed data (square rooted) | | Z=x-y | Z² |
|----|----------------|--------------|-------------|-------------|-------|-----|
| | | | Culture (x) | Rapid (y) | | |
| 170 | 2 | 2 | 1.4 | 1.4 | 0 | 0 |
| 171 1 | 2 | 2 | 1.4 | 1.4 | 0 | 0 |
| 172 | 1 | 1 | 1 | 1 | 0 | 0 |
| 173 | 1 | 1 | 1 | 1 | 0 | 0 |
| 174 | 1 | 1 | 1 | 1 | 0 | 0 |
| 175 | 1 | 1 | 1 | 1 | 0 | 0 |
| 176 | 2 | 2 | 1.4 | 1.4 | 0 | 0 |
| 177 | 1 | 1 | 1 | 1 | 0 | 0 |
| 178 | 1 | 1 | 1 | 1 | 0 | 0 |
| 179 | 1 | 1 | 1 | 1 | 0 | 0 |
| 180 | 1 | 1 | 1 | 1 | 0 | 0 |
| 181 1 | 1 | 1 | 1 | 1 | 0 | 0 |
| 182 | 1 | 1 | 1 | 1 | 0 | 0 |
| 183 | 1 | 1 | 1 | 1 | 0 | 0 |
| 184 | 1 | 1 | 1 | 1 | 0 | 0 |
| 185 | 2 | 2 | 1.4 | 1.4 | 0 | 0 |
| 186 | 1 | 1 | 1 | 1 | 0 | 0 |
| 187 | 1 | 1 | 1 | 1 | 0 | 0 |
| 188 | 1 | 1 | 1 | 1 | 0 | 0 |
| 189 | 1 | 1 | 1 | 1 | 0 | 0 |
| 190 | 1 | 1 | 1 | 1 | 0 | 0 |
| 191 | 1 | 1 | 1 | 1 | 0 | 0 |
| 192 | 1 | 1 | 1 | 1 | 0 | 0 |
| 193 | 1 | 1 | 1 | 1 | 0 | 0 |
| 194 | 1 | 1 | 1 | 1 | 0 | 0 |
| 195 | 1 | 1 | 1 | 1 | 0 | 0 |
| 196 | 1 | 1 | 1 | 1 | 0 | 0 |
| 197 | 1 | 1 | 1 | 1 | 0 | 0 |
| 198 | 1 | 1 | 1 | 1 | 0 | 0 |
| 199 | 1 | 1 | 1 | 1 | 0 | 0 |
| 200 | 1 | 1 | 1 | 1 | 0 | 0 |
| 201 | 1 | 1 | 1 | 1 | 0 | 0 |
| 202 | 1 | 1 | 1 | 1 | 0 | 0 |
| 203 | 1 | 1 | 1 | 1 | 0 | 0 |
| 205 | 1 | 1 | 1 | 1 | 0 | 0 |

Table 18: Rapid* *Legionella* Comparative Analysis
*The 'rapid' method is the method of the present invention

(continued)

| Table 18: Rapid* *Legionella* Comparative Analysis *The 'rapid' method is the method of the present invention | | | | | | |
|---|---|---|---|---|---|---|
| No | Culture Result | Rapid Result | Transformed data (square rooted) | | Z=x-y | Z² |
| | | | Culture (x) | Rapid (y) | | |
| 206 | 1 | 1 | 1 | 1 | 0 | 0 |
| 207 | 1 | 1 | 1 | 1 | 0 | 0 |
| 208 | 1 | 1 | 1 | 1 | 0 | 0 |
| 209 | 1 | 1 | 1 | 1 | 0 | 0 |
| 210 | 1 | 1 | 1 | 1 | 0 | 0 |
| 211 1 | 1 | 1 | 1 | 1 | 0 | 0 |
| 212 | 1 | 1 | 1 | 1 | 0 | 0 |
| 213 | 1 | 1 | 1 | 1 | 0 | 0 |
| 214 | 1 | 1 | 1 | 1 | 0 | 0 |
| 215 | 1 | 2 | 1 | 1.4 | 0.4 | 0.16 |
| 216 | 1 | 1 | 1 | 1 | 0 | 0 |
| 217 | 1 | 1 | 1 | 1 | 0 | 0 |
| 218 | 1 | 1 | 1 | 1 | 0 | 0 |
| 219 | 1 | 1 | 1 | 1 | 0 | 0 |
| 220 | 1 | 1 | 1 | 1 | 0 | 0 |
| 221 | 1 | 1 | 1 | 1 | 0 | 0 |
| 222 | 1 | 1 | 1 | 1 | 0 | 0 |
| 223 | 1 | 1 | 1 | 1 | 0 | 0 |
| 224 | 1 | 1 | 1 | 1 | 0 | 0 |
| 225 | 1 | 1 | 1 | 1 | 0 | 0 |
| 226 | 1 | 1 | 1 | 1 | 0 | 0 |
| 227 | 1 | 1 | 1 | 1 | 0 | 0 |
| 228 | 2 | 2 | 1.4 | 1.4 | 0 | 0 |
| 229 | 1 | 1 | 1 | 1 | 0 | 0 |
| 230 | 1 | 1 | 1 | 1 | 0 | 0 |
| 231 | 2 | 2 | 1.4 | 1.4 | 0 | 0 |
| 232 | 1 | 1 | 1 | 1 | 0 | 0 |
| 233 | 1 | 1 | 1 | 1 | 0 | 0 |
| 234 | 1 | 2 | 1 | 1.4 | 0.4 | 0.16 |
| 235 | 1 | 1 | 1 | 1 | 0 | 0 |
| 236 | 1 | 1 | 1 | 1 | 0 | 0 |
| 237 | 1 | 1 | 1 | 1 | 0 | 0 |
| 238 | 1 | 1 | 1 | 1 | 0 | 0 |
| 239 | 1 | 1 | 1 | 1 | 0 | 0 |
| 240 | 1 | 1 | 1 | 1 | 0 | 0 |

(continued)

| No | Culture Result | Rapid Result | Transformed data (square rooted) | | Z=x-y | Z$^2$ |
|---|---|---|---|---|---|---|
| | | | Culture (x) | Rapid (y) | | |
| 241 | 1 | 1 | 1 | 1 | 0 | 0 |
| 242 | 1 | 1 | 1 | 1 | 0 | 0 |
| 243 | 1 | 1 | 1 | 1 | 0 | 0 |
| 244 | 1 | 1 | 1 | 1 | 0 | 0 |
| 245 | 1 | 1 | 1 | 1 | 0 | 0 |
| 246 | 1 | 1 | 1 | 1 | 0 | 0 |
| 247 | 1 | 1 | 1 | 1 | 0 | 0 |
| 248 | 1 | 1 | 1 | 1 | 0 | 0 |
| 249 | 1 | 1 | 1 | 1 | 0 | 0 |
| 250 | 1 | 1 | 1 | 1 | 0 | 0 |
| 251 | 1 | 1 | 1 | 1 | 0 | 0 |
| 252 | 1 | 1 | 1 | 1 | 0 | 0 |
| 253 | 1 | 1 | 1 | 1 | 0 | 0 |
| 254 | 1 | 1 | 1 | 1 | 0 | 0 |
| 255 | 1 | 1 | 1 | 1 | 0 | 0 |
| 256 | 1 | 1 | 1 | 1 | 0 | 0 |
| 257 | 1 | 1 | 1 | 1 | 0 | 0 |
| 258 | 1 | 1 | 1 | 1 | 0 | 0 |
| 259 | 1 | 1 | 1 | 1 | 0 | 0 |
| 260 | 1 | 1 | 1 | 1 | 0 | 0 |
| 261 | 1 | 1 | 1 | 1 | 0 | 0 |
| 262 | 1 | 1 | 1 | 1 | 0 | 0 |
| 263 | 1 | 1 | 1 | 1 | 0 | 0 |
| 264 | 1 | 1 | 1 | 1 | 0 | 0 |
| 265 | 1 | 1 | 1 | 1 | 0 | 0 |
| 266 | 1 | 1 | 1 | 1 | 0 | 0 |
| 267 | 1 | 1 | 1 | 1 | 0 | 0 |
| 268 | 1 | 1 | 1 | 1 | 0 | 1 |
| 269 | 2 | 2 | 1.4 | 1.4 | 0 | 0 |
| 270 | 1 | 1 | 1 | 1 | 0 | 1 |
| 271 | 1 | 1 | 1 | 1 | 0 | 0 |
| 272 | 1 | 1 | 1 | 1 | 0 | 0 |
| 273 | 1 | 1 | 1 | 1 | 0 | 0 |
| 274 | 1 | 1 | 1 | 1 | 0 | 0 |
| 275 | 2 | 2 | 1.4 | 1.4 | 0 | 0 |

Table 18: Rapid* *Legionella* Comparative Analysis
*The 'rapid' method is the method of the present invention

(continued)

| No | Culture Result | Rapid Result | Transformed data (square rooted) | | Z=x-y | Z² |
|---|---|---|---|---|---|---|
| | | | Culture (x) | Rapid (y) | | |
| 276 | 1 | 1 | 1 | 1 | 0 | 0 |
| 277 | 2 | 2 | 1.4 | 1.4 | 0 | 0 |
| 278 | 1 | 1 | 1 | 1 | 0 | 0 |
| 279 | 1 | 1 | 1 | 1 | 0 | 0 |
| 280 | 1 | 1 | 1 | 1 | 0 | 0 |
| 281 | 1 | 1 | 1 | 1 | 0 | 0 |
| 282 | 1 | 1 | 1 | 1 | 0 | 0 |
| 283 | 1 | 1 | 1 | 1 | 0 | 0 |
| 284 | 1 | 1 | 1 | 1 | 0 | 0 |
| 285 | 1 | 1 | 1 | 1 | 0 | 0 |
| 286 | 1 | 1 | 1 | 1 | 0 | 0 |
| 287 | 1 | 1 | 1 | 1 | 0 | 0 |
| 288 | 1 | 1 | 1 | 1 | 0 | 0 |
| 289 | 1 | 1 | 1 | 1 | 0 | 0 |
| 290 | 1 | 1 | 1 | 1 | 0 | 0 |
| 291 1 | 1 | 1 | 1 | 1 | 0 | 0 |
| 292 | 1 | 1 | 1 | 1 | 0 | 0 |
| 293 | 1 | 1 | 1 | 1 | 0 | 0 |
| 294 | 1 | 1 | 1 | 1 | 0 | 0 |
| 295 | 1 | 1 | 1 | 1 | 0 | 0 |
| 296 | 1 | 1 | 1 | 1 | 0 | 0 |
| 297 | 1 | 1 | 1 | 1 | 0 | 0 |
| 298 | 1 | 1 | 1 | 1 | 0 | 0 |
| 299 | 1 | 1 | 1 | 1 | 0 | 0 |
| 300 | 1 | 1 | 1 | 1 | 0 | 0 |
| 301 | 1 | 1 | 1 | 1 | 0 | 0 |
| 302 | 1 | 1 | 1 | 1 | 0 | 0 |
| 303 | 1 | 1 | 1 | 1 | 0 | |
| 304 | 1 | 1 | 1 | 1 | 0 | 0 |
| 305 | 1 | 1 | 1 | 1 | 0 | 0 |
| 306 | 1 | 1 | 1 | 1 | 0 | 0 |
| 307 | 1 | 1 | 1 | 1 | 0 | 0 |
| 308 | 1 | 1 | 1 | 1 | 0 | 0 |
| 309 | 1 | 1 | 1 | 1 | 0 | 0 |
| 310 | 1 | 1 | 1 | 1 | 0 | 0 |

Table 18: Rapid* *Legionella* Comparative Analysis
*The 'rapid' method is the method of the present invention

(continued)

| No | Culture Result | Rapid Result | Transformed data (square rooted) | | Z=x-y | Z² |
|----|----------------|--------------|-------------|-----------|-------|-----|
| | | | Culture (x) | Rapid (y) | | |
| 311 | 1 | 1 | 1 | 1 | 0 | 0 |

Table 18: Rapid* *Legionella* Comparative Analysis — *The 'rapid' method is the method of the present invention

Statistical analysis:

$$n = 311 \qquad\qquad \Sigma z = 4.8 \qquad\qquad \Sigma z^2 = 1.92$$
$$\text{Mean of } z = 0.015$$
$$\frac{(\Sigma z)^2}{n} = 0.07$$

$$\text{Variance of } z = \frac{1.92 - 0.07}{n - 1} = s^2_z \qquad s^2_z = 0.006 \qquad s_z = 0.08$$

$$\text{Variance about mean of } z = 4.8 \pm 1.96 \times \frac{0.08}{\sqrt{310}} = 4 \pm 0.009$$

1.96 is taken from t-tables for ∞ (120+) degrees of freedom (n-1) and a probability of 5 *i.e.* there is a 95% confidence that both methods will give the same result.

To test the null hypothesis that the difference in the data sets is zero calculate:

$$1.96 = \frac{0.015}{0.08/\sqrt{311}} = 3$$

The null hypothesis is rejected if the result is greater than t (1.96)

In this instance the hypothesis that the two data sets are from the same population is rejected, i.e. statistically these two data sets are from different population. With the rapid test identifying more positives than the culture method.

In addition to the above two further analyses were conducted on the pairs of data.

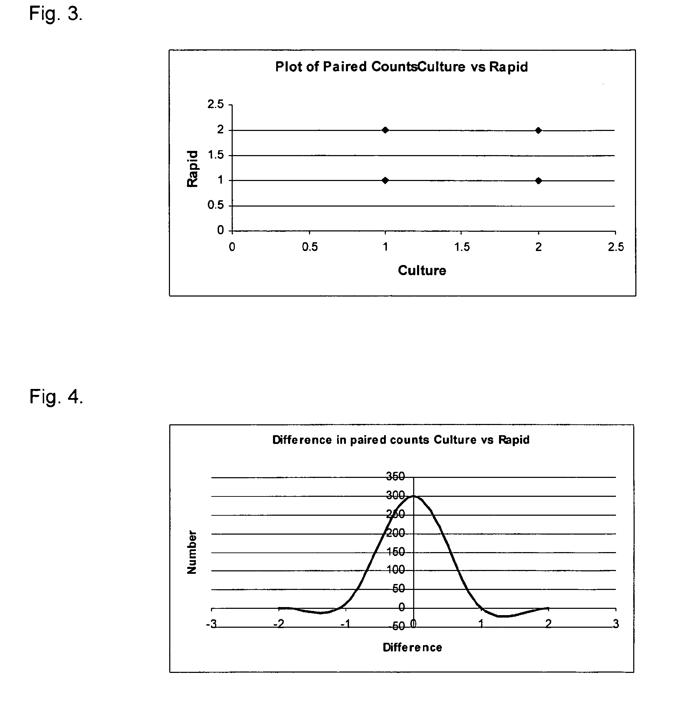

Firstly the difference in the paired counts was illustrated graphically by plotting the paired data (Fig. 3). The presence/absence of *Legionella* by the culture method is plotted on the x-axis against the presence/absence of *Legionella* by the method of the present invention which is plotted on the y-axis. A score of 2 indicates a *Legionella* positive result and a score of 1 indicates *Legionella* negative result.

The plot of Fig. 3 appears to indicate that both methods will perform equally well in determining both positive and negative samples. However it also appears to show that in some cases the culture method will be positive and the method of the present invention negative and in other cases the reverse is true. If the raw data is examined it is evident that this is not the case, and is illustrated in Fig. 4.

Here the difference in the recovery rates was calculated, and the number of each difference was ranked and plotted.

Fig. 4 shows that a difference of 0 occurs on 120 occasions out of 130. This shows that in the vast majority of cases there was no difference between the results obtained from the two methods.

<u>References</u>

1. Fields, B.S., Benson, R.F. and Besser, R.E. (2002) Clinical Microbiology Reviews. Vol 15(3): 506-526. Legionella and Legionnaire's Disease: 25 Years of Investigation.
2. Roig, J., and Rello, J. (2003) Journal of Antimicrobial Chemotherapy. Vol 51: 1119-1129 Legionnaires' disease: a rational approach to therapy.
3. Szymanska, J. (2004) Annals of Agricultural and Environmental Medicine. Vol 11: 9-12. Risk of exposure to Legionella in dental practice.

**Claims**

1.   A method for detecting *Legionella* comprising:

(a) obtaining a sample of liquid suspected of containing *Legionella,*
(b) concentrating the sample to produce a concentrated sample, and
(c) staining an aliquot of the concentrated sample with *Legionella-specific* stain.

2.   A method according to claim 1, wherein the concentration step of the method comprises two concentration steps.

3.   A method according to claim 2, comprising the steps of (i) filtering *Legionella* from the sample of liquid with a filter (ii) eluting *Legionella* from the filter and (iii) concentrating *Legionella* in the eluent by centrifugation.

4.   A method according to any preceding claim wherein the stain comprises labelled *Legionella-specific* antibodies.

5.   A method according to claim 4 wherein the antibodies are monoclonal antibodies.

6.   A method according to any preceding claim wherein the stain comprises fluorescein isothiocyanate (FITC) labelled antibodies.

7.   A method according to any of claims 1 to 6, wherein the stain is specific for *Legionella pneumophila.*

8.   A method according to claim 7 wherein the stain is specific for *Legionella pneumophila* serogroup 1.

9.   A method according to claim 7, wherein the stain is specific for the group comprising serogroups 1 to 15 of *Legionella pneumophila.*

10.  A method according to any preceding claim wherein a first aliquot of the sample is stained with a stain specific for serogroup 1 of *Legionella pneumophila* and a second aliquot of the sample is stained with a stain specific for the group comprising serogroups 1 to 15 of *Legionella*

11.  A method according to any preceding claim, further comprising:

(d) staining an aliquot of the sample with a viability stain.

12.  A method according to claim 11, wherein the viability stain is selected from propidium iodide, acridine orange and methylene blue.

13.  A method according to any preceding claim wherein a 1000-fold to 5000-fold concentration is achieved.

14.  A method according to any preceding claim wherein an aliquot of the sample is incubated under conditions suitable for culturing *Legionella.*

**15.** A kit for detecting *Legionella* by a method according to any preceding claim comprising (i) a filter for filtering *Legionella* from a sample of liquid and (ii) a *Legionella*-specific stain.

**16.** A kit according to claim 15 wherein the stain is specific for *Legionella pneumophila* serogroup 1.

**17.** A kit according to claim 15 or 16 wherein the stain is specific for the group comprising serogroups 1 to 15 of *Legionella pneumophila.*

**18.** A kit according to any of claims 15 to 17 wherein a first aliquot of the sample is stained with a stain specific for serogroup 1 of *Legionella pneumophila* and a second aliquot of the sample is stained with a stain specific for the group comprising serogroups 1 to 15 of *Legionella.*

**19.** A kit according to any of claims 16 to 18 further comprising a viability stain.

**20.** A kit according to claim 19 wherein the viability stain is selected from propidium iodide, acridine orange and methylene blue.

**21.** A kit according to claim 20 further comprising a plate for culturing bacteria.

**22.** A kit according to any of claims 15 to 21 further comprising a *Legionella* sample.

Fig. 1.

```
┌─────────────────────────────────┐
│          1 litre Water          │
└─────────────────────────────────┘
                 ↓
┌─────────────────────────────────┐
│      Filter through membrane    │
└─────────────────────────────────┘
                 ↓
┌─────────────────────────────────┐
│  Release bacteria from membrane │
│     and resuspend to 20 ml      │
└─────────────────────────────────┘
```

A    B    C

| Treat ⅓ sample with acid | Treat ⅓ sample with heat | Leave ⅓ sample untreated |
|---|---|---|
| Plate 0.5 ml onto selective agar | Plate 0.5 ml onto selective agar | Plate 0.5 ml onto selective agar |

Incubate plates (36 °C, 10 Days)

Read plates at days 4, 7 and 10

Pick off potential *Legionella* colonies and carry out serological identification

14 days

Fig. 2.

```
                    ┌─────────────────────────────┐
                    │        1 litre Water         │
                    └─────────────────────────────┘
                                  │
                                  ▼
                    ┌─────────────────────────────┐
                    │    Filter through membrane   │
                    └─────────────────────────────┘
                                  │
                                  ▼
                    ┌─────────────────────────────┐
                    │  Release bacteria from filter and  │
                    │       resuspend to 10 ml     │
                    └─────────────────────────────┘
                                  │
                                  ▼
                    ┌─────────────────────────────┐
                    │  Centrifuge and resuspend in 1 ml  │
                    └─────────────────────────────┘
                                  │
                                  ▼
                    ┌─────────────────────────────┐
                    │ Centrifuge and resuspend in 0.3 ml │
                    └─────────────────────────────┘
```

A                       B                       C

| Stain 0.1 ml for Serogroup 1 | Stain 0.1 ml for Serogroups 1 - 15 | Plate 0.1 ml onto Agar |

| Counterstain with viability stain | Counterstain with viability stain | |

4 hours

Fig. 3.

**Plot of Paired Counts Culture vs Rapid**

Fig. 4.

**Difference in paired counts Culture vs Rapid**

**European Patent Office**

**EUROPEAN SEARCH REPORT**

Application Number

EP 08 25 0438

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 01/40505 A (ULP-CETRE D'ANALYSES ET DE RECHERCHES.) 7 June 2001 (2001-06-07) | 1-4,7-9, 14-17 | INV. C12Q1/04 G01N33/569 |
| Y | * page 5, line 8 - line 31 * | 5,6, 10-13, 18-22 | |
| | * page 12, line 22 - page 13, line 20 * * claims 12,13 * ----- | | |
| Y | WO 01/42791 A (BINAX INC.) 14 June 2001 (2001-06-14) * claims 1-4,8 * ----- | 10,18 | |
| Y | DELGADO-VISCOGLIOSI PILAR ET AL: "Rapid method for enumeration of viable Legionella pneumophila and other Legionella spp. in water" APPLIED AND ENVIRONMENTAL MICROBIOLOGY, vol. 71, no. 7, July 2005 (2005-07), pages 4086-4096, XP002480308 ISSN: 0099-2240 *page 4087, column 1, last paragraph before "Materials and Methods".* *page 4087 -4088, section "Materials and Methods".* ----- | 6,10-13, 18-22 | |
| Y | US 4 931 547 A (P. HOFFMAN ET AL.) 5 June 1990 (1990-06-05) * column 6, line 1 - line 15; claims 1,2 * ----- | 5 | |

TECHNICAL FIELDS SEARCHED (IPC)

C12Q
G01N

The present search report has been drawn up for all claims

| Place of search | Date of completion of the search | Examiner |
|---|---|---|
| The Hague | 16 May 2008 | Griffith, Gerard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 08 25 0438

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2008

| Patent document cited in search report | | Publication date | Patent family member(s) | | Publication date |
|---|---|---|---|---|---|
| WO 0140505 | A | 07-06-2001 | AU | 2181501 A | 12-06-2001 |
| | | | FR | 2801677 A1 | 01-06-2001 |
| WO 0142791 | A | 14-06-2001 | AU | 783777 B2 | 01-12-2005 |
| | | | AU | 2429301 A | 18-06-2001 |
| | | | CA | 2393628 A1 | 14-06-2001 |
| | | | EP | 1234183 A1 | 28-08-2002 |
| US 4931547 | A | 05-06-1990 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FIELDS, B.S. ; BENSON, R.F. ; BESSER, R.E.** Legionella and Legionnaire's Disease: 25 Years of Investigation. *Clinical Microbiology Reviews,* 2002, vol. 15 (3), 506-526

- **ROIG, J. ; RELLO, J.** Legionnaires' disease: a rational approach to therapy. *Journal of Antimicrobial Chemotherapy,* 2003, vol. 51, 1119-1129
- **SZYMANSKA, J.** Risk of exposure to Legionella in dental practice. *Annals of Agricultural and Environmental Medicine,* 2004, vol. 11, 9-12